# EUROPEAN PATENT SPECIFICATION

(11) **EP 0 585 206 B1**
(45) Date of publication and mention of the grant of the patent: **08.12.1999**
(21) Application number: 93830269.2
(22) Date of filing: 24.06.1993
(51) Int. Cl.: C07D 209/16, A61K 31/40

(54) **Tryptamines for treatment of circadian rhythm disorders**
Tryptamine zur Behandlung von Störungen der circadianen Rhythmik
Tryptamines pour traitement des troubles du rythme circadien

(30) Priority: 01.07.1992 IT MI921612
(43) Date of publication of application: 02.03.1994
(73) Proprietor: I.F.L.O. S.a.s. di Giorgio e Aldo Laguzzi, I-20127 Milano (IT)
(72) Inventor: Duranti, Ermanno, I.F.L.O. S.a.s. di Giorgio e, I-20127 Milano (IT); Fraschini, Franco, I.F.L.O. S.a.s. di Giorgio e, I-20127 Milano (IT); Laguzzi, Aldo, I.F.L.O. S.a.s. di Giorgio e, I-20127 Milano (IT); Stankov, Bojidar, I-20129 Milano (IT)
(74) Representative: Cicogna, Franco

(56) References cited:
- EP-A- 0 126 630
- EP-A- 0 483 077
- EP-A- 0 513 702
- WO-A-89/01472
- MOLECULAR PHARMACOLOGY vol. 42, no. 4, 1992, pages 619 - 626 R. J. MOUREY ET AL. '(3H)Noscapine Binding Sites in Brain: relationship to Indoleamines and the Phosphoinositide and Adenylyl Cyclase Messenger Systems'
- ARCH. PHARM. vol. 304, no. 7, 1971, WEINHEIM pages 523 - 531 F. EIDEN, U. KUCKL[NDER '3-Aethenyl-5-methoxy-indol-Derivate'

## Description

The present invention relates to synthetic pharmaceutical preparations and novel compounds, having a tryptamine nucleus, particularly effective in pathologies which are related to the circadian rhythms.

### DESCRIPTION OF THE PRIOR ART

Under conditions of natural environment, the endogenous biological rhythms are adapted to the regular alternation of day and night and to the slower cycles (months and seasons). The human seasonality is clearly perceptible and under certain circumstances extremely important (for instance Seasonal Affective Disorder, or winter depression), in spite of the fact that particularly in the developed countries, artificial heating and lighting are universally used.

Man has remained a diurnal species and the current social organization frequently causes a lack of correspondence between the body physiology and the environment.

N-Acetyl-5-methoxytryptamine is a hormone secreted rhythmically by the pineal gland of vertebrates, with particularly high concentrations in the pineal, the cerebrospinal fluid and in the peripheral blood during nighttime.

The synthesis and secretion of N-acetyl-5-methoxytryptamine by the pineal gland are characterized by a circadian rhythm, controlled by an endogenous biological clock.

This rhythm has a phase entrained to the photoperiod by means of light. In view of this fact, N-acetyl-5-methoxytryptamine is considered one of the principal transducers of the photoperiodic information from the environment.

In fact, the most well known function of this hormone is that of influencing the reproductive activity of animals which are highly photoperiodic in terms of their reproductive competence.

Studies carried out by the applicant in the last few years have permitted to synthesize, characterize and evaluate a number of compounds which bind with high affinity to the specific N-acetyl-5-methoxytryptamine receptors in the central nervous system, and which are capable of producing biological effects.

The document WO-A-8901472 describes melatonin analogues which are substantially lacking the -OCH₃ group at C-5 (see pp 7-8; examples 1,3,4,5). All compounds are substituted at C-2 position indirectly, i.e. all possess at least one -CH₂- moiety (m = 1 to 3) between C-2 and the R¹ (See Title, Claim 1 and elsewhere in this prior document).

It is therefore possible to assume that these new tryptaminic derivatives can be employed for treatment of pathologies related to circadian disorganization and disease (chronopathology).

Particularly unexpected and most surprising is the fact that, depending upon the substituents on the ring and on the lateral chain, tryptaminic derivatives which have an agonist or an antagonist action, with an activity superior 10-30 times with respect to the basic molecule, can be obtained.

There are reported hereinbelow, examples of the synthesis with a reaction scheme of some of these substances as well as their evaluation.

The reaction scheme is as follows:

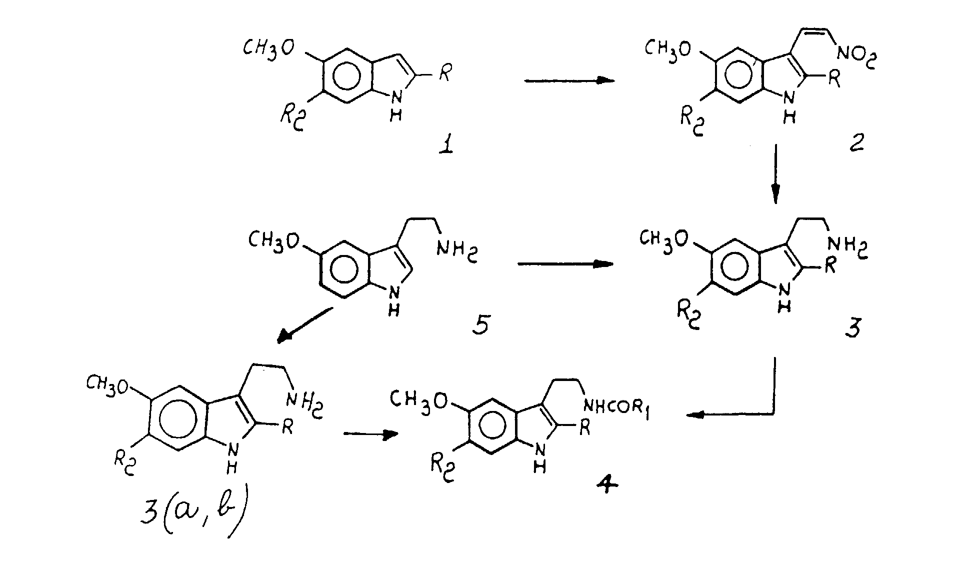

### NITRO-OLEFINATION OF 5-METHOXY 2-SUBSTITUTED INDOLES (2 a-c)

To a solution of 1-dimethylamino-2-nitroethylene (1,16 g., 10 mM) in trifluoroacetic acid (TFA) (6 cc) cooled to 0°C is added a 5-methoxy 2-substituted indole (10 mM).

The reaction mixture is stirred at room temperature under nitrogen for about 0.5 hour. It is then poured in ice-water and is extracted with ethyl acetate or methylene chloride.

The organic solvent is removed under reduced pressure and the residue is purified by crystallization.

### PREPARATION OF 5-METHOXY 2-SUBSTITUTED TRYPTAMINES (3)

To a suspension of LiAlH₄ (1,14 g, 30 mM) in anhydrous tetrahydrofuran (THF) (30 cc) under nitrogen is slowly added a solution of 3-(2-nitrovinyl-5-methoxy 2-substituted indoles (2,5mM) in anhydrous tetrahydrofuran (THF) (30 cc) and the mixture is refluxed for about 45 minutes. After cooling there is carefully added water to destroy the excess of the lithium aluminum hydride.

The mixture is filtered on Celite® and the filtrate is concentrated under reduced pressure.

The residual aqueous phase is evaporated under reduced pressure and the crude product is used for the subsequent steps.

### PREPARATION OF N-ACYL-2, AND 2,6-SUBSTITUTED-5-METHOXY TRYPTAMINES (4 a-1)

To a cold solution of crude 2 or 2,6-substituted 5-methoxytryptamine (3; 3a,b) (4 mM) and triethanolamine (TEA) (4 mM) in anhydrous tetrahydrofuran (12 cc) is added the new acylating compound (acetic anhydride) (4 mM) and the mixture so obtained is kept stirring at room temperature for about 15 hours. The solvent is then evaporated and the residue is dissolved in ethyl acetate and washed with a saturated bicarbonate solution. The organic phase is distilled under reduced pressure and the crude product is purified by flash chromatography (ethyl acetate-cyclohexane 7:3).

All the substances were evaluted for their relative affinity and were tested for biological activity by using classical tests.

Supplied herein are the chemical-physical data of the tryptamine analogs which are shown in Table I

**TABLE I**

| Composition | R | R₁ | R₂ | Yield (%) | m.p. | Solvent for Recrystallization |
|---|---|---|---|---|---|---|
| 2a | CH₃ | -- | H | 58 | -- | CH₂ Cl₂ - hexane |
| 2b | C₆ H₁₁ | -- | H | 60 | 114° | CH₂ Cl₂ - hexane |
| 2c | C₆ H₅ | -- | H | 79 | 170° | CH₂ Cl₂ - hexane |
| 3a | Br | -- | H | 60 | 136-138° | EtOAc - hexane |
| 3b | Br | -- | Br | 50 | 146-147° | EtOAc - hexane |
| 4a | CH₃ | CH₃ | H | 50 | 122° | EtOAc - hexane |
| 4b | C₃ H₇ | CH₃ | H | 30 | amorphous | -- |
| 4c | C₆ H₁₁ | CH₃ | H | 51 | amorphous | -- |
| 4d | C₆ H₅ | CH₃ | H | 52 | -- | MeOH-ET₂O |
| 4e | CH₃ | cyclopropyl | H | 44 | -- | EtOAc - hexane |
| 4f | CH₃ H₇ | cyclopropyl | H | 25 | 142-143° | EtOAc - hexane |
| 4g | C₆ H₅ | cyclopropyl | H | 60 | amorphous | -- |
| 4h | Br | cyclopropyl | H | 25 | subl 85° | CH₂ Cl₂ - hexane |
| 4i | I | cyclopropyl | H | 30 | subl 87° | CH₂ Cl₂ - hexane |
| 4j | Br | CH₃ | H | 35 | 141-142° | EtOAc - hexane |
| 4k | I | CH₃ | H | 40 | 146-149° | EtOAc - hexane |
| 4l | Br | CH₃ | Br | 30 | 146-148* | EtOAc - hexane |

The compounds exhibit the properties shown in Table 2.

**TABLE 2**

| Compound | Ka ± SE (L/mole) | | | ki (1/Ka) (moles/L) | Activity |
|---|---|---|---|---|---|
| 4a | 2.3E⁺⁹ | ± | 7.7E⁺⁸ | 4.33E⁻¹⁰ | Agonist |
| 4b | 2.4E⁺⁸ | ± | 1.7E⁺⁷ | 4.30E⁻⁹ | Agonist |
| 4c | 1.9E⁺⁸ | ± | 7.1E⁺⁷ | 5.30E⁻⁹ | Partial antagonist |
| | | | | | |
| 4d | 1.8E⁺¹⁰ | ± | 5.6^{E+9} | 5.70E⁻¹¹ | Antagonist |
| 4e | 1.5E⁺⁹ | ± | 4.1E⁺⁸ | 6.30E⁻¹⁰ | Agonist |
| 4f | 5.5E⁺⁷ | ± | 7.1E⁺⁶ | 1.8 E⁻⁸ | No Activity |
| 4g | 4.1E+9 | ± | 5.1E+8 | 2.40E⁻¹⁰ | Partial antagonist |
| | | | | | |
| 4h | 4.6E⁺⁸ | ± | 2.2E⁺⁷ | 2.10E⁻¹⁰ | Agonsit |
| 4i | 1.0E⁺¹⁰ | ± | 2.1E⁺⁹ | 1.0E⁻¹⁰ | Agonist |
| 4j | 1.7E⁺¹⁰ | ± | 5.9E⁺⁹ | 5.80E⁻¹¹ | Agonist |
| 4k | 4.6E⁺¹⁰ | ± | 5.9E⁺⁹ | 2.10E⁻¹¹ | Agonist |
| 4l | 1.5E⁺¹⁰ | ± | 3.1E⁺⁹ | 6.7E⁻¹¹ | Agonist |
| Melatonin | 6.5E | ± | 7.1E⁺⁷ | 1.5E⁻⁹ | Agonist |

Clinical application of the agonists.
1) Disorders of the circadian organization.
   1.a jet-lag.
      The most evident circadian desynchronization is observed in individuals who cross rapidly more than 5 time zones, particularly in the eastern direction.
   1.b Disturbances of sleep such as disorders of the temporal macro- and microstructure of sleep, psychophysiological insomnia and the "Delayed Phase Sleep Syndrone".
      The new tryptamine derivatives synthesized by the applicant such as for instance N-acetyl-2-bromo-5-methoxy tryptamine may substitute the therapy of the association of N-acetyl-5-methoxytryptamine with benzodiazepines (BDZ) in cases of sleep disturbances. We refer to the substances which are characterized with the name agonist. The benzodiazepines administered at night, cause phase delays of the endogenous circadian clock with a consequent interruption of the circadian rhythms.
      This action is due, in addition to their effect at the neuronal thalamocortical network, also to a direct influence on the internal biological clock (the suprachiasmatic nuclei, SCN).
      In cases of sleep disturbances, very frequently, also the endogenous rhythm of melatonin, the biological marker of the circadian pacemaker function, is altered.
      The benzodiazepines exhibit side effects such as diurnal anxiety and suicidal ideations.
      High density of N-acetyl-5-metoxytryptamine receptors,binding the new tryptaminic derivatives with high affinity, are found in the SCN of mammals, including man.
      Melatonin, administrered similtaneously with a benzodiazepine, causes a phase reinforcement, thus compensating the deleterious benzodiazepine effect on the circadian clock.
      The new substances synthesized by the applicant, by themselves are in condition to improve the temporal organization of sleep, influencing the macro- and the microstructure through interaction with the gamma-amino-butyric acid receptor complex (GA- Boa).
   1.c People working "on shifts"
      The shift-workers frequently suffer disturbances of the circadian rhythm which manifest themselves with out-of-phase sleep-wake cycles, insomnia, diurnal hypoactivity and depression. The endogenous rhythm of melatonin is phase-shifted.
   1.d Treatment of desynchronized blind people
      Totally blind people frequently suffer from disturbances connected to their state of "free-running" circadian rhythmicity.
2. Aging
   With the advance of age, the concentrations of melatonin both in the serum and the pineal gland decrease as a consequence of the degeneration of the pineal. A number of problems connected with the dysfunction of the gland in the elderly propably should be considered as an alteration of the circadian rhythyms, partially connected also to an altered sensitivity to light.
   Main application are:
   2.a. Correction of tne alterations of the circadian rhythms (described hereinabove)
   2.b. Sleep disturbances (described hereinabove).
   2.c. Immune deficit.

   In the last few years, the immunomodulating properties of N-acetyl-5-methoxytryptamine have been demonstrated using several in vivo and in vitro models.
   The novel agonists synthesized by the applicant are capable of inducing chemotaxis of human mononuclear cells, isolated from peripheral blood, showing a direct involvement in the transmission of the cell-to-cell signal in the immune system.
3 Neoplasms
   The direct action of the new agonists in reducing the cellular proliferation has been demonstrated in vitro in a few model systems (human melanoma and murine melanoma, mammary carcinoma cell lines).
   The in vivo studies carried out in several species have shown that the new agonist compounds may be used in combined treatment with chemotherapy. The in vivo action can also be due to their positive influence on the immune system.
4) Control of Ovulation
   It has been demonstrated that the new tryp-tamines may be used in combination with low doses of progestin compounds, in order to control the menstrual cycle and to prevent ovulation, and conception, in very low doses (10-30 mg) dose/per day.

### CLINICAL APPLICATION OF THE ANTAGONISTS

1) Seasonal affective disorder (SAD)
   The phase delay observed in the majority of patients affected by SAD has been corrected by means of exposing them in the morning hours to bright light, which causes phase advance and is lowering the high levels of endogenous melatonin. In these patients, the rhythm of melatonin is abnormal. Therefore, the potent N-acetyl-5-methoxytryptamine antagonists could be therapeutically used for treatment during the day, in order to correct the phase-delay, and to avoid the effects of endogenous melatonin.
2) Treatment of hypothalmic amenorrhea in women.
   A great number of women in the reproductive age, suffer from this type of amenorrhea, connected to abnormally high levels of endogenous N-acetyl-5-methoxytryptamine in the peripheral blood.
3) Treatment of animals which exhibit seasonality in the reproductive cycles.
   For the commercial breeding, it is important that the births of animals occur in limited periods of the year and well defined periods of time. Animals which mate in time of the year with a longer period of light such as, for instance, the horse (long day breeders) may be treated with the antagonists of the new compounds during the short days of winter in order to induce reproductive activity. On the contrary, the short day breeders such as, for instance the sheep, may be treated with the agonists of the new compounds during the long days of summer in order to induce gonadal activity.

The compounds thus conceived may be subjects to several variations and modifications which all fall within the scope of the present invention. In addition, all the details related to the administration as well as the doses may be varied as a function of each individual and/or patient, always within the scope of the present invention.

## Claims

1. Synthetic pharmacological compounds of the tryptamine type particularly effective in the treatment of pathologies related to circadian rhythm disorders, and having the general formula: 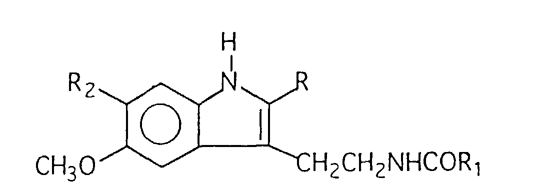 the compounds being listed in the Table below:
| Compound No. | R | R₁ | R₂ |
|---|---|---|---|
| 4b | C₃H₇ | CH₃ | H |
| 4c | C₆H₁₁ | CH₃ | H |
| 4e | CH₃ | cyclopropyl | H |
| 4f | C₃H₇ | cyclopropyl | H |
| 4g | C₆H₅ | cyclopropyl | H |
| 4h | Br | cyclopropyl | H |
| 4l | Br | CH₃ | Br |
| 4m | I | cyclopropyl | H |

2. A pharmaceutical composition which contains as the active ingredient a compound of the formula hereinbelow: 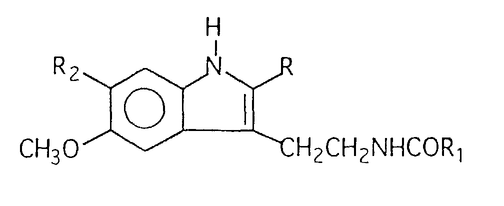 wherein:
R = C₃H₇
R₁ = CH₃
R₂ = H

3. A pharmaceutical composition which contains as the active ingredient a compound of the formula hereinbelow: 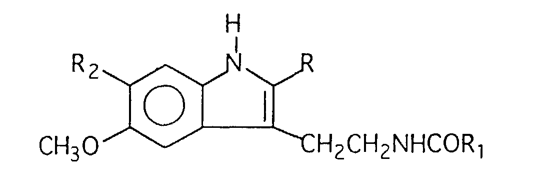 wherein:
R = CH₃
R₁ = CH₃
R₂ = H

4. A pharmaceutical composition which contains as the active ingredient a compound of the formula hereinbelow: 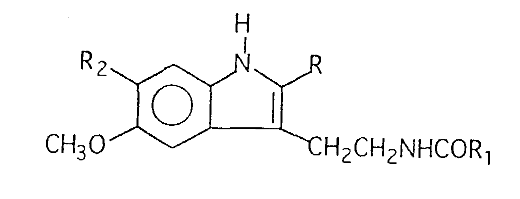 wherein:
R = C₆H₁₁
R₁ = CH₃
R₂ = H

5. A pharmaceutical composition which contains as the active ingredient a compound of the formula hereinbelow: 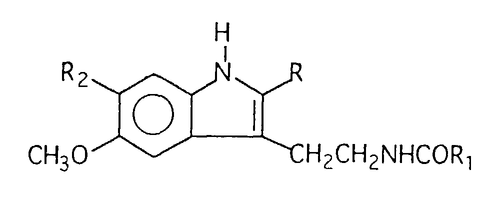 wherein:
R = C₆H₅
R₁ = CH₃
R₂ = H

6. A pharmaceutical composition which contains as the active ingredient a compound of the formula hereinbelow: 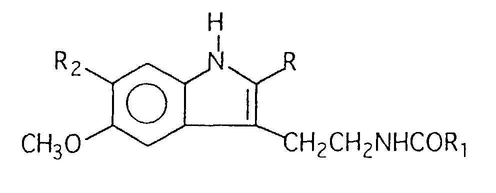 wherein:
R = CH₃
R₁ = cyclopropyl
R₂ = H

7. A pharmaceutical composition which contains as the active ingredient a compound of the formula hereinbelow: 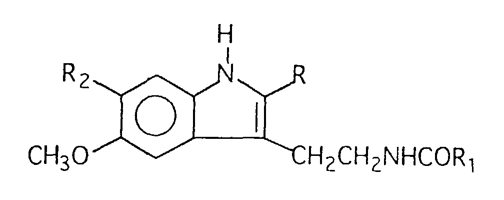 wherein:
R = C₆H₅
R₁ = cyclopropyl
R₂ = H

8. A pharmaceutical composition which contains as the active ingredient a compound of the formula hereinbelow: 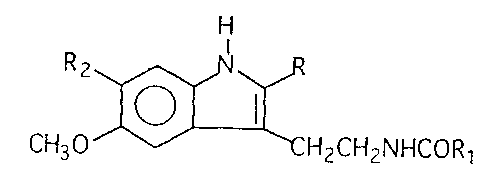 wherein:
R = Br
R₁ = cyclopropyl
R₂ = H

9. A pharmaceutical composition which contains as the active ingredient a compound of the formula hereinbelow: 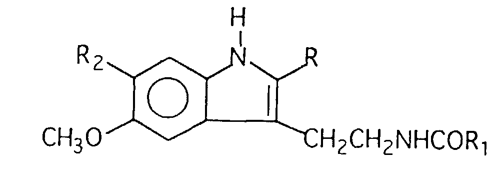 wherein:
R = I
R₁ = cyclopropyl
R₂ = H

10. A pharmaceutical composition which contains as the active ingredient a compound of the formula hereinbelow: 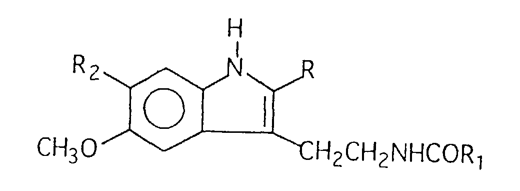 wherein:
R = Br
R₁ = CH₃
R₂ = H

11. A pharmaceutical composition which contains as the active ingredient a compound of the formula hereinbelow: 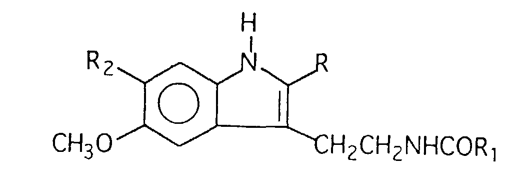 wherein:
R = I
R₁ = CH₃
R₂ = H

12. A pharmaceutical composition which contains as the active ingredient a compound of the formula hereinbelow: 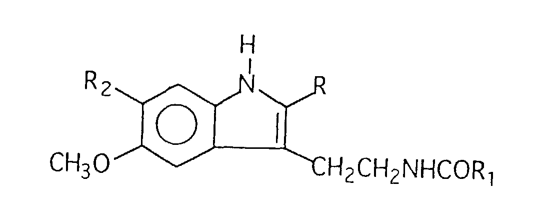 wherein:
R = Br
R₁ = CH₃
R₂ = Br

13. A pharmaceutical composition which contains at least one of the compounds according to claims 2-12, said compound being an agonist, clinically useful for the therapy of circadian rhythm disorders or chronopathology, whereby circadian desynchronization which is present in subjects rapidly crossing several time zones is eliminated.

14. A pharmaceutical composition which contains at least one of the compounds according to claims 2-12, said compound being an agonist, clinically useful for the therapy of sleep disturbances in human subjects and for the therapy of blind people who are desynchronized.

15. A pharmaceutical composition which contains at least one of the compounds according to claims 2-12, said compound being clinically useful for the treatment of specific pathologies connected with advance of age and in the treatment of subjects exhibiting alterations of the circadian rhythms, sleep disturbances and immune deficit.

16. A pharmaceutical composition which contains at least one of the compounds according to claims 2-12, said compound being an antagonist, useful for the therapy of Seasonal Affective Disorders, for treatment of animals that exhibit seasonality in the reproductive cycle, and for treatment of patients suffering from hypothalamic amenorrhea.

17. A pharmaceutical composition which contains at least one of the compounds according to claims 2-12, wherein said compound is effective in treatment of pathologies which interfere with the circadian rhythms.

## Patentansprüche

1. Synthetische Verbindungen vom Tryptamintyp mit pharmakologischen Eigenschaften, die bei der Behandlung von Krankheitsbildern, die in Zusammenhang mit Störungen des zirkadianen Rhythmus stehen, besonders wirksam sind und die allgemeine Formel besitzen: 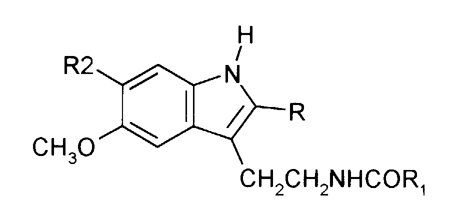 wobei die Verbindungen in der nachstehenden Tabelle aufgeführt sind:
| Verbindung Nr. | R | R1 | R2 |
|---|---|---|---|
| 4b | C₃H₇ | CH₃ | H |
| 4c | C₆H₁₁ | CH₃ | H |
| 4e | CH₃ | Cyclopropyl | H |
| 4f | C₃H₇ | Cyclopropyl | H |
| 4g | C₆H₅ | Cyclopropyl | H |
| 4h | Br | Cyclopropyl | H |
| 41 | Br | CH₃ | Br |
| 4m | | Cyclopropyl | H |

2. Eine pharmazeutische Zusammensetzung, die als aktiven Bestandteil eine Verbindung der nachstehenden Formel besitzt: 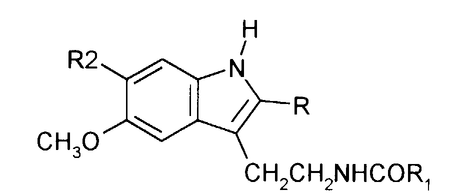 worin:
R = C₃H₇
R1 = CH₃
R2 = H

3. Eine pharmazeutische Zusammensetzung, die als aktiven Bestandteil eine Verbindung der nachstehenden Formel besitzt: 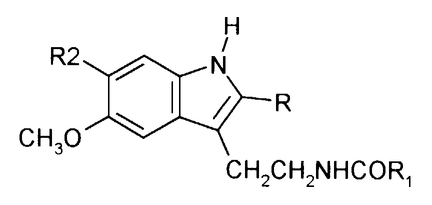 worin:
R = CH₃
R1 = CH₃
R2 = H

4. Eine pharmazeutische Zusammensetzung, die als aktiven Bestandteil eine Verbindung der nachstehenden Formel besitzt: 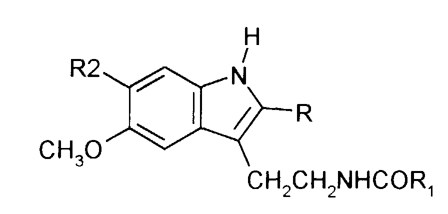 worin:
R = C₆H₁₁
R1 = CH₃
R2 = H

5. Eine pharmazeutische Zusammensetzung, die als aktiven Bestandteil eine Verbindung der nachstehenden Formel besitzt: 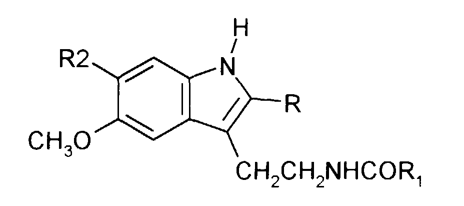 worin:
R = C₆H₅
R1 = CH₃
R2 = H

6. Eine pharmazeutische Zusammensetzung, die als aktiven Bestandteil eine Verbindung der nachstehenden Formel besitzt: 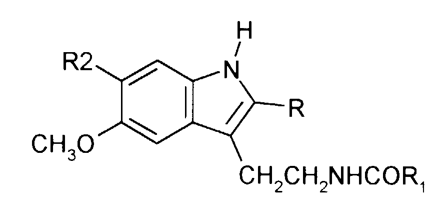 worin:
R = CH₃
R1 = Cyclopropyl
R2 = H

7. Eine pharmazeutische Zusammensetzung, die als aktiven Bestandteil eine Verbindung der nachstehenden Formel besitzt: 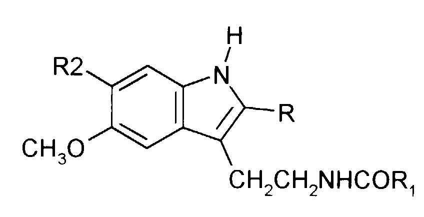 worin:
R = C₆H₅
R1 = Cyclopropyl
R2 = H

8. Eine pharmazeutische Zusammensetzung, die als aktiven Bestandteil eine Verbindung der nachstehenden Formel besitzt: 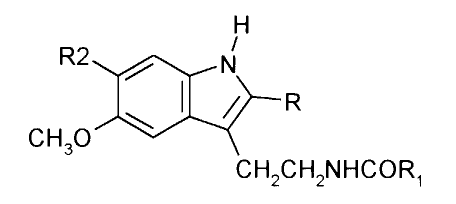 worin:
R = Br
R1 = Cyclopropyl
R2 = H

9. Eine pharmazeutische Zusammensetzung, die als aktiven Bestandteil eine Verbindung der nachstehenden Formel besitzt: 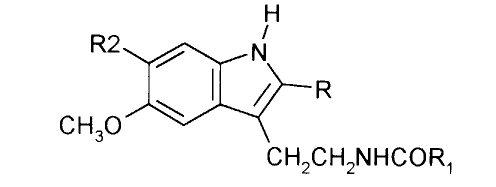 worin:
R = I
R1 = Cyclopropyl
R2 = H

10. Eine pharmazeutische Zusammensetzung, die als aktiven Bestandteil eine Verbindung der nachstehenden Formel besitzt: 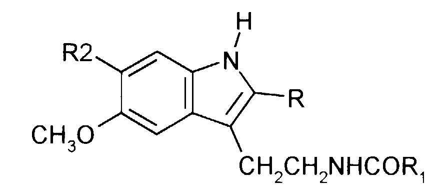 worin:
R = Br
R1 = CH₃
R2 = H

11. , Eine pharmazeutische Zusammensetzung, die als aktiven Bestandteil eine Verbindung der nachstehenden Formel besitzt: 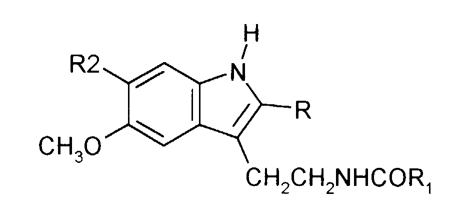 worin:
R = I
R1 = CH₃
R2 = H

12. Eine pharmazeutische Zusammensetzung, die als aktiven Bestandteil eine Verbindung der nachstehenden Formel besitzt: 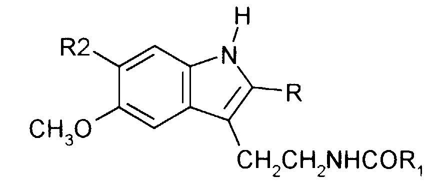 worin:
R = Br
R1 = CH₃
R2 = Br

13. Eine pharmazeutische Zusammensetzung, die mindestens eine der Verbindungen nach den Ansprüchen 2-12 enthält, wobei diese Verbindung ein für die Therapie der Störungen des zirkadianen Rhythmus oder einer Chronopathologie klinisch nützlicher Agonist ist, durch den eine zirkadiane Desynchronisierung, wie sie bei Individuen auftritt, die rasch mehrere Zeitzonen durchlaufen, beseitigt wird.

14. Eine pharmazeutische Zusammensetzung, die mindestens eine der Verbindungen nach den Ansprüchen 2-12 enthält, wobei diese Verbindung ein für die Therapie von Schlafstörungen des Menschen und von desynchronisierten, blinden Menschen klinisch nützlicher Agonist ist.

15. Eine pharmazeutische Zusammensetzung, die mindestens eine der Verbindungen nach den Ansprüchen 2-12 enthält, wobei diese Verbindung für die Behandlung spezieller, mit dem Altern einhergehender Krankheitsbilder klinisch nützlich ist. sowie bei der Behandlung von Individuen, die Veränderungen im zirkadianen Rhythmus, Schlafstörungen und ein Immundefizit aufweisen.

16. Eine pharmazeutische Zusammensetzung, die mindestens eine der Verbindungen nach den Ansprüchen 2-12 enthält, wobei diese Verbindung ein für die Therapie saisonabhängiger Gemütsstörungen klinisch nützlicher Antagonist ist, insbesondere für die Behandlung von Tieren, deren Reproduktionszyklus von der Jahreszeit abhängt, und für die Behandlung von Patientinnen, die unter hypothalamischer Amenorrhoe leiden.

17. Eine pharmazeutische Zusammensetzung, die mindestens eine der Verbindungen nach den Ansprüchen 2-12 enthält, worin diese Verbindung bei der Behandlung von Krankheitsbildern nützlich ist, die mit dem zirkadianen Rhythmus interferieren.

## Revendications

1. Composés pharmacologiques synthétiques de type tryptamine particulièrement efficaces dans le traitement de pathologies relatif aux désordres du rythme circadien, et présentant la formule générale : 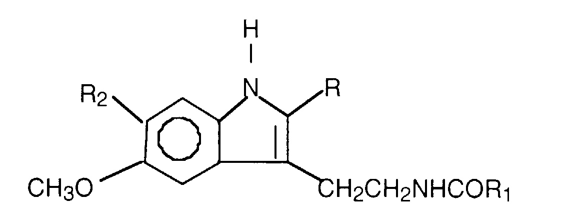 le composé étant listé dans le tableau ci-dessous :
| Composé n° | R | R₁ | R₂ |
|---|---|---|---|
| 4b | C₃H₇ | CH₃ | H |
| 4c | C₆H₁₁ | CH₃ | H |
| 4e | CH₃ | cyclopropyle | H |
| 4f | C₃H₇ | cyclopropyle | H |
| 4g | C₆H₅ | cyclopropyle | H |
| 4h | Br | cyclopropyle | H |
| 4l | Br | CH₃ | Br |
| 4m | | cyclopropyle | H |

2. Composition pharmaceutique qui contient comme ingrédient actif un composé de formule ci-dessous : 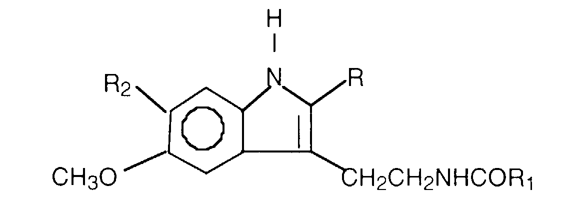 dans lequel:
R = C₃H₇
R₁ = CH₃
R₂ = H

3. Composition pharmaceutique qui contient comme ingrédient actif un composé de formule ci-dessous : 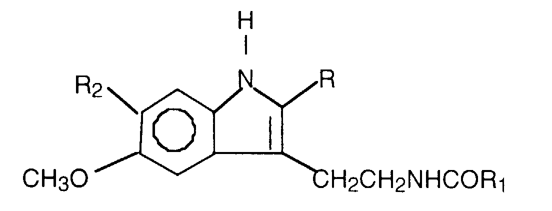 dans lequel:
R = CH₃
R₁ = CH₃
R₂ = H

4. Composition pharmaceutique qui contient comme ingrédient actif un composé de formule ci-dessous : 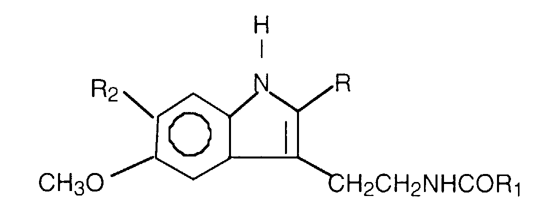 dans lequel :
R = C₆H₁₁
R₁ = CH₃
R₂ = H

5. Composition pharmaceutique qui contient comme ingrédient actif un composé de formule ci-dessous : 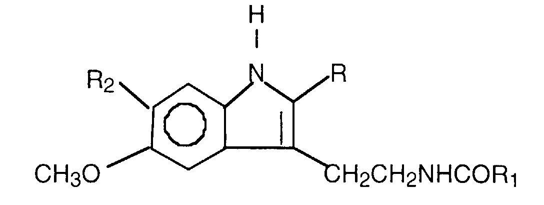 dans lequel :
R = C₆H₅
R₁ = CH₃
R₂ = H

6. Composition pharmaceutique qui contient comme ingrédient actif un composé de formule ci-dessous : 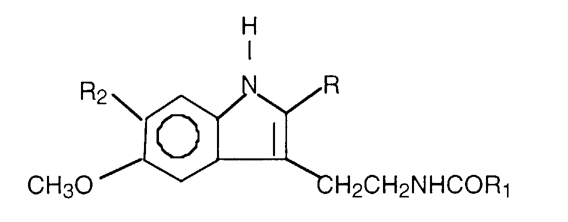 dans lequel :
R = CH₃
R₁ = cyclopropyle
R₂ = H

7. Composition pharmaceutique qui contient comme ingrédient actif un composé de formule ci-dessous : 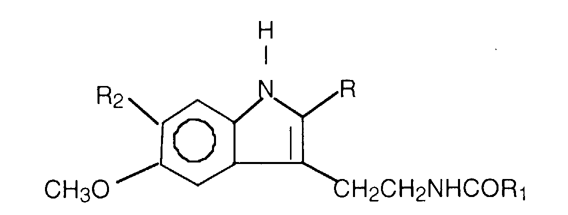 dans lequel :
R = C₆H₅
R₁ = cyclopropyle
R₂ = H

8. Composition pharmaceutique qui contient comme ingrédient actif un composé de formule ci-dessous : 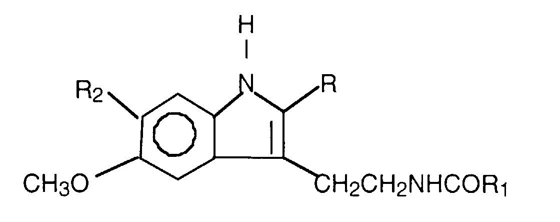 dans lequel :
R = Br
R₁ = cyclopropyle
R₂ = H

9. Composition pharmaceutique qui contient comme ingrédient actif un composé de formule ci-dessous : 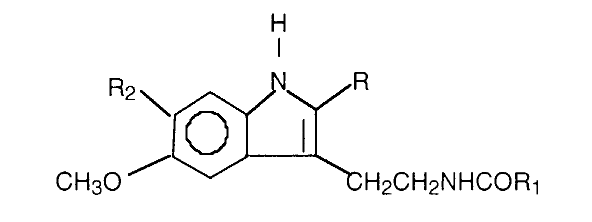 dans lequel :
R = I
R₁ = cyclopropyle
R₂ = H

10. Composition pharmaceutique qui contient comme ingrédient actif un composé de formule ci-dessous : 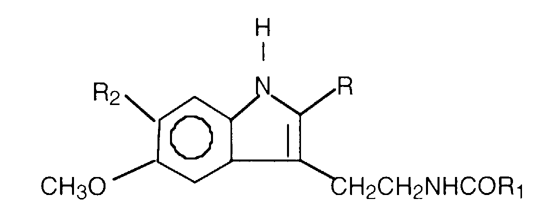 dans lequel :
R = Br
R₁ = CH₃
R₂ = H

11. Composition pharmaceutique qui contient comme ingrédient actif un composé de formule ci-dessous : 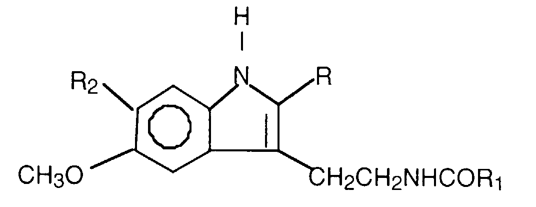 dans lequel :
R = I
R₁ = CH₃
R₂ = H

12. Composition pharmaceutique qui contient comme ingrédient actif un composé de formule ci-dessous : 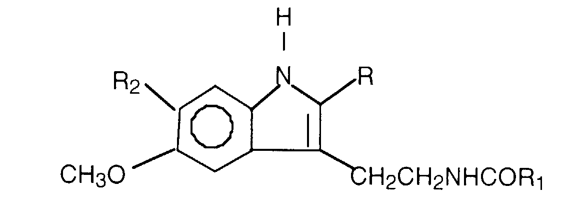 dans lequel:
R = Br
R₁ = CH₃
R₂ = Br

13. Composition pharmaceutique qui contient au moins un des composés selon les revendications 2 à 12, ledit composé étant un agoniste, cliniquement utile pour le traitement des désordres du rythme circadien ou à d'une chronopathologie, où une désynchronisation circadienne, qui est présente chez des sujets traversant plusieurs périodes de temps rapidement, est éliminée.

14. Composition pharmaceutique qui contient au moins un des composés selon les revendications 2 à 12, ledit composé étant un agoniste, cliniquement utile pour le traitement des perturbations du sommeil chez des sujets humains ou pour la thérapie de personnes aveugles qui sont désynchronisées.

15. Composition pharmaceutique qui contient au moins un des composés selon les revendications 2 à 12, ledit composé étant cliniquement utile pour le traitement de pathologiques spécifiques liées avec le vieillissement et dans le traitement de sujets montrant des altérations du rythme circadien, des perturbations du sommeil et d'un déficit immunitaire.

16. Composition pharmaceutique qui contient au moins un des composés selon les revendications 2 à 12, ledit composé étant un antagoniste, utile pour le traitement de désordres affectifs saisonniers, pour le traitement d'animaux montrant un saisonnalité dans le cycle reproductif, et pour le traitement de patients souffrant d'aménorrhées hypothalamiques.

17. Composition pharmaceutique qui contient au moins un des composés selon les revendications 2 à 12, dans laquelle ledit composé est efficace pour le traitement de pathologies qui interfère avec les rythmes circadien.
